# EUROPÄISCHE PATENTSCHRIFT

(11) **EP 1 131 668 B1**
(45) Veröffentlichungstag und Bekanntmachung des Hinweises auf die Patenterteilung: **02.05.2002**
(21) Anmeldenummer: 99955875.2
(22) Anmeldetag: 11.11.1999
(51) Int. Cl.: G02C 7/12, A61F 9/02

(54) **SKIBRILLE ZUR VERBESSERUNG DER RÄUMLICHEN ORIENTIERUNG**
SKI GOGGLES FOR IMPROVING SPATIAL ORIENTATION
LUNETTES DE SKI PERMETTANT UNE MEILLEURE ORIENTATION SPATIALE

(30) Priorität: 18.11.1998 DE 19853133
(43) Veröffentlichungstag der Anmeldung: 12.09.2001
(73) Patentinhaber: PREUSSNER, Paul Rolf, Dr., 55131 Mainz (DE)
(72) Erfinder: PREUSSNER, Paul Rolf, Dr., 55131 Mainz (DE)
(86) Internationale Anmeldenummer: EP9908033
(87) Internationale Veröffentlichungsnummer: WO0029898

(56) Entgegenhaltungen:
- WO-A-94/08266
- DE-A- 3 346 627
- US-A- 2 440 104
- US-A- 4 431 265
- US-A- 4 989 274
- US-A- 5 414 476

## Beschreibung

### Technisches Gebiet

Bei der Erfindung handelt es sich um eine Skibrille, mit der der Träger seine räumliche Orientierung während des Skifahrens verbessern kann.

### Stand der Technik

Skibrillen, wie sie dem Stand der Technik entsprechen, haben den Zweck, ihren Träger vor zu starker, schädlicher Sonnenstrahlung zu schützen. Außerdem gibt es winddichte Ausführungen, deren Gehäuse dem Gesicht vollständig anliegt, und deren Frontscheibe aus einer durchgehenden, biegsamen Kunststoffplatte besteht. Darüberhinaus sind Ausführungen bekannt, die bei schlechten Sichtverhältnissen den subjektiven Seheindruck des Trägers verbessern sollen. Dies ist speziell beim Abfahrtsskifahren (einschließlich verwandter Sportarten, wie z.B. Snowboard- oder Schlittenfahren) wichtig, weil die verschneite oder vereiste Geländeoberfläche bei insgesamt sehr hoher Reflektivität nur geringe Unterschiede in der Richtungsabhängigkeit der Reflektivität aufweist. Unterschiede in der Geländeneigung sind daher nur bei Sonnenschein genügend gut erkennbar. Bereits bei mäßig bewölktem Himmel, ansonsten aber freier Sicht, ist die Erkennbarkeit der Geländestrukturen soweit reduziert, daß das Skifahren hierdurch limitiert ist, und die Sturzgefahr deutlich ansteigt.

Zur Verbesserung des Sehens beim Skifahren sind verschiedene optische Mittel bekannt geworden, in deren Beschreibung meist der Begriff "Kontrast" verwendet wird. Dieser Begriff wird aber uneinheitlich gebraucht. Meist wird implizit unterstellt, daß es einen "nützlichen" Anteil des Lichtes gibt, der die gewünschte Information trägt, und einen "störenden" der nur das Auge mehr oder weniger blendet, und die "Kontrastverbesserung" dann darin besteht, daß ein Teil der störenden Strahlung herausgefiltert wird.

Im einfachsten Fall werden farbselektive Filter verwendet, wobei unterstellt wird, daß das Auge in den herausgefilterten Farben stärker als in den duchgelassenen geblendet wird. Diese Maßnahme ist aber nur dann sinnvoll, wenn die Blendung von Streulicht herrührt, das durch die Streuung an Partikeln entsteht, die *nicht* auf der Geländeoberfläche liegen, sondern sich in der Luft zwischen dieser und dem Betrachter befinden (z.B. Nebeltröpfchen, Schneeflocken), und der Streukoeffizient für Streuung an diesen Partikeln von der Wellenlänge abhängt, was auch nur schwach der Fall ist.

In DE2649842 wird eine Brille beschrieben, die mittels Polarisationsfiltern linear polarisiertes Licht herausfiltert, das von ebenen Wasseroberflächen ins Auge gelangt und dieses dann stark blendet, wenn eine Lichtquelle, vor allem die Sonne, spiegelnd abgebildet wird. Dieser Effekt ist relevant an Gewässeroberflächen bei Sonnenschein oder an regennassen Straßenoberflächen, an denen das Licht von Scheinwerfern von Fahrzeugen gespiegelt wird, nicht aber beim Skifahren bei bewölktem Himmel.

In EP0582800 wird eine Skibrille beschrieben, die eine Richtungsfilterung vornimmt. Sie kann eine "Kontrastverbesserung" wieder nur dann bewirken, wenn das "störende" Licht aus Streulicht besteht, z.B. bei Nebel oder Schneefall. Das gesunde menschliche Auge wird ansonsten durch seitlich einfallendes Licht grundsätzlich dann nicht geblendet, wenn dieses nicht wesentlich heller als das von vorne kommende ist. Bei bewölktem Himmel und ansonsten klarer Sicht fällt von der Seite kein helleres Licht ins Auge als von der Schneefläche von vorne.

Eine "Kontrastverbesserung" mittels optischen Filtern, gleich welcher Art, ist bei bewölktem Himmel und klarer Sicht im Schneegelände nicht möglich und insofern nicht geeignet, die optische Orientierung des Skifahrers zu verbessern, weil das von der Geländeoberfläche ins Auge fallende Licht nicht aus "nützlichen" und "störenden" Anteilen besteht bzw. in solche zerlegbar ist.

Die optische Orientierung des Menschen im dreidimensionalen Gelände hängt aber nicht nur ab von der Wahrnehmung von Helligkeits- und / oder Farbunterschieden der Geländeoberfläche. Vielmehr entsteht der dreidimensionale Seheindruck durch mehrere, sich ergänzende monokular und binokular wahrnehmbare Effekte.

Zum monokularen Raumeindruck trägt beispielsweise bei (und wird seit Jahrhunderten in der Malerei benutzt), daß bereits eine geringe Anisotropie in der Ausleuchtung, wie sie praktisch immer gegeben ist, diejenige Seite einer gewölbten Oberfläche am hellsten erscheinen läßt, deren Flächennormale die Winkelhalbierende des Winkels ist, den der auf die Oberfläche gerichtete Sehstrahl und die Richtung, in der das Ausleuchtungslicht am hellsten ist, miteinander bilden (Spiegelbedingung). Dieser Effekt beruht auf der Richtungsabhängigkeit des Reflektionskoeffizienten der meisten Materialoberflächen. Das reflektierte Licht ist zu einem großen Teil polarisiert ("natürliche Polarisation").

Zum binokularen Seheindruck trägt unter anderem die sogenannte Querdisparation bei, die darauf beruht, daß die geometrische Abbildung des selben Sehobjektes in beide Augen zu unterschiedlichen Projektionsbildern führt. Da sich dieser Effekt geometrisch berechnen läßt, können dreidimensionale Bilder beispielsweise dadurch simuliert werden, daß man den Seheindruck für das rechte Auge in linear polarisiertem Licht in einer Ebene erzeugt, den für das linke in der dazu senkrechten Ebene, und dann eine Brille aufsetzt wie aus DE2649842, Anspruch 4, bekannt, bei der dem rechten und dem linken Auge die entsprechenden Polarisationsfilter vorgesetzt werden, deren Polarisationsebenen aufeinander senkrecht stehen.

In US5,414,476 wird vorgeschlagen, mit der aus DE2649842 bekannten Brille die oben erwähnte "natürliche Polarisation" zur Beurteilung von Oberflächen zu verwenden. Durch Rotation der beiden Gläser soll deren Polarisationsebene je nach Situation so gedreht werden, daß ein möglichst großer Unterschied zwischen dem Seheindruck des rechten und des linken Auges entsteht. Nach Meinung der Autoren soll dadurch auch eine mögliche Dominanz eines Auges korrigiert werden können.

**Aufgabe der vorliegenden Erfindung** ist es, eine Skibrille verfügbar zu machen, die Unterschiede in der Geländeneigung der verschneiten oder vereisten Oberfläche auch bei bewölktem Himmel besser erkennen läßt, wobei die speziellen Eigenschaften und Fähigkeiten gerade des beidäugigen menschlichen Sehvermögens mit genutzt werden sollen.

### Kurze Darstellung der Erfindung

Die Aufgabe wird erfindungsgemäß entsprechend den Merkmalen des Anspruchs 1 gelöst. Den beiden Augen werden Polarisationsfilter vorgesetzt, deren Polarisationsebenen aufeinander senkrecht stehen, und die mit der Horizontalen einen Winkel von 45° bzw. 135° bilden. Brillen mit Polarisationsfiltern, deren Polarisationsebenen aufeinander senkrecht stehen, sind für einen ganz anderen Zweck, nämlich zur Betrachtung synthetischer Stereobilder, ansonsten bekannt, beispielsweise aus US4,431,265. Die Wirkung der hier für den Einsatz beim Skifahren vorgeschlagenen Brille ist aber eine völlig andere. Sie ist folgendermaßen zu verstehen.

Die Geländeoberfläche bewirkt eine Reflektion, die in verschiedenen Polarisationsrichtungen unterschiedlich stark ist. Verschiedene Geländeneigungen reflektieren jeweils einen etwas höheren oder etwas geringeren Anteil an Licht in 45° oder 135° Polarisation. Betrachtet man nun mit einem Auge den 45°-Anteil, mit dem anderen den 135°-Anteil, so erscheint einem Auge eine Geländeneigung etwas dunkler, dem anderen etwas heller als eine benachbarte Region, für eine andere Geländeposition ist es umgekehrt. Die Polarisation wird also hier als zusätzlicher, dem menschlichen Auge sonst nicht zugänglicher Informationsträger verwendet. Das visuelle System des Menschen reagiert hochempfindlich auf geringste Unterschiede im Seheindruck zwischen beiden Augen und versucht, diese in die Beurteilung des dreidimensionalen Raumeindrucks einzubeziehen, auch dann, wenn ihnen keine geometrisch eindeutige Querdisparation entspricht. Subjektiv ergibt sich aus den wahrgenommenen Helligkeitsunterschieden zwischen beiden Augen eine erhebliche Steigerung dessen, was anschaulich als "Plastizität" bezeichnet wird.

Würde man einem Auge die horizontale, dem anderen die vertikale Polarisationsrichtung anbieten, so entstünde ein derart unnatürlicher Seheindruck, daß eine sportliche Bewegung mit einer solchen Brille nicht mehr möglich wäre, denn ein Auge sähe vor allem horizontale Flächen drastisch dunkler als das andere.

### Bevorzugte Ausführung und Ausführungsalternativen

In ihrer einfachsten Ausführung besteht die genannte Skibrille aus einem Brillengestell in irgendeiner der hierfür üblicherweise verwendeten Ausführungen mit Brillengläsern aus Glas oder Kunststoff, die mit irgendeiner der hierfür bekannten Techniken als Polarisationsfilter ausgeführt sind.

Da solche Filter meist eine Gesamttransmission zwischen 40% und 50% aufweisen, wirkt die Brille ohnehin bereits als "Sonnenbrille" mit mäßiger Lichtabschwächung.

In einer anderen Ausführung der Erfindung ist die Absorption im optischen und / oder ultravioletten Bereich zusätzlich soweit erhöht, wie es bei Sonnenbrillen, speziell Skibrillen, üblicherweise der Fall ist.

In einer weiteren Ausführung der Erfindung sind die Gläser zusätzlich sphärisch und / oder zylindrisch so geschliffen, daß sie den Refraktionsfehler des Trägers korrigieren.

In einer weiteren Ausführung der Erfindung sind die Polarisationsfilter in die Frontscheibe einer Skibrille integriert, wie sie üblicherweise als dem Stand der Technik entsprechende, geschlossene Skibrille mit gleichzeitiger Windschutzfunktion bekannt ist. In dieser Ausführung kann beispielsweise polarisierende Folie auf die biegsame Frontscheibe der Skibrille aufgebracht sein, oder diese Scheibe ist bereits aus polarisierendem Material gefertigt.

## Patentansprüche

1. Verwendung einer Polarisationsbrille als Skibrille, die für ihren Träger Unterschiede in der Neigung der verschneiten oder vereisten Geländeoberfläche dadurch besser erkennbar werden läßt, daß das von dieser Geländeoberfläche reflektierte, teilweise linear polarisierte Licht dem rechten und dem linken Auge durch getrennte Polarisationsfilter zugeführt wird, wobei die Polarisationsebenen dieser beiden Filter aufeinander senkrecht stehen und mit der Horizontalen einen Winkel von 45° bzw. 135° bilden, wodurch subjektiv für den Träger der Skibrille Unterschiede in der Polarisation als Unterschiede in der Helligkeit erscheinen, die den Eindruck erhöhter "Plastizität" hervorrufen.

2. Verwendung nach Anspruch 1, wobei die Polarisationsfilter gleichzeitig als Brillengläser zur Korrektur des Refraktionsfehlers des Trägers ausgebildet sind.

3. Verwendung nach Anspruch 1, wobei die Polarisationsfilter auf eine durchgehende Frontscheibe aufgebracht oder in diese integriert sind, wie sie für geschlossene Windschutz-Skibrillen in bekannter Technik verwendet werden.

4. Verwendung nach Anspruch 1, wobei die optische Absorption über die Polarisation hinaus soweit verstärkt ist, wie es für Sonnenschutzgläser (Sonnenbrillen) erforderlich ist.

## Claims

1. Use of polarisation eye glasses as ski eyeglasses which allow differences in the slope of snow-covered or ice-covered terrain surfaces to become better recognisable for their wearer by the fact that the reflected, partially linearly polarised light from this terrain surface is led to the right and the left eye through separate polarisation filters, whereby the polarisation planes of these two filters are perpendicular to one another forming an angle of 45° and 135° respectively with the horizontal, as a result of which for the wearer of the ski eyeglasses differences in the polarisation appear as differences in the brightness which induce the impression of increased "plasticity".

2. Use in accordance with claim 1, whereby the polarisation filters are finished simultaneously as spectacle lenses for the correction of the wearer's error of refraction.

3. Use in accordance with claim 1, whereby the polarisation filters are mounted on a continuous front shield or integrated into the same, as they are used for closed wind-proof ski goggles in the known technique.

4. Use in accordance with claim 1, whereby the optical absorption is intensified beyond the polarisation so far as is required for solar protection glasses (sunglasses).

## Revendications

1. Utilisation de lunettes de polarisation en tant que lunettes de ski qui permettent au sujet qui les porte de mieux reconnaître les différences de déclivité de la surface enneigée ou glacée du relief du fait que cette lumière, qui-est reflétée par la surface du terrain et en partie polarisée de manière linéaire, est transmise à l'oil droit et à l'oil gauche par des filtres de polarisation séparés, où les plans de polarisation de ces deux filtres sont placés verticalement l'un sur l'autre et constituent avec l'horizontale un angle de 45° ou de 135°, de manière que le sujet porteur des lunettes de ski ait subjectivement l'impression que les différences de polarisation sont des différences de luminosité qui créent une impression de «plasticité»renforcée.

2. Utilisation selon la revendication 1, où les filtres de polarisation sont en même temps conçus comme des verres de lunettes pour la correction du défaut de réfraction du porteur.

3. Utilisation selon la revendication 1, où les filtres de polarisation sont placés sur un masque monopièce ou intégrés à celui-ci, comme cela se fait selon une technique bien connue pour les lunettes de ski enveloppantes.

4. Utilisation selon la revendication 1, où l'absorption optique, au-delà de la polarisation, est renforcée autant qu'il est nécessaire pour des verres de protection solaire (lunettes de soleil).
